# FASCICULE DE BREVET EUROPEEN

(11) **EP 3 927 292 B1**
(45) Date de publication et mention de la délivrance du brevet: **05.04.2023**
(21) Numéro de dépôt: 20710232.8
(22) Date de dépôt: 17.02.2020
(51) Int. Cl.: A61F 5/56

(54) **DISPOSITIF DE RÉGLAGE, MESURE, ENREGISTREMENT DES RAPPORTS D'OCCLUSION EN PROTRUSION MANDIBULAIRE, APPLICATION À UN APPAREIL D'IMMOBILISATION EN PROTRUSION MANDIBULAIRE**
EINSTELLEN DES GERÄTS, MESSEN, AUFZEICHNEN VON MANDIBULÄREN SCHUTZBERICHTEN, ANWENDUNG AUF MANDIBULÄRE PROTRUSION IMMOBILISIERUNGSGERÄTE
ADJUSTING DEVICE, MEASUREMENT, RECORDING OF MANDIBULAR PROTRUSION OCCLUSION REPORTS, APPLICATION TO MANDIBULAR PROTRUSION IMMOBILIZATION APPARATUS

(30) Priorité: 18.02.2019 FR 1901618; 18.02.2019 FR 1901619; 08.11.2019 FR 1912576
(43) Date de publication de la demande: 29.12.2021
(73) Titulaire: Botbol, Charles, 75008 Paris (FR)
(72) Inventeur: Botbol, Charles, 75008 Paris (FR)
(74) Mandataire: Jacobacci Coralis Harle
(86) Numéro de dépôt international: PCT/FR2020/050284
(87) Numéro de publication internationale: WO 2020/169909

(56) Documents cités:
- EP-A1- 2 491 901
- EP-A2- 1 838 259
- EP-B1- 1 838 259
- WO-A1-2018/096295
- US-A- 5 829 441
- US-A- 5 868 138
- US-A1- 2013 333 707
- US-A1- 2015 360 115

## Description

### Domaine technique

La présente invention concerne de manière générale le domaine des appareils buccodentaires. Elle concerne plus particulièrement un dispositif de réglage, de mesure et d'enregistrement des rapports d'occlusion en protrusion mandibulaire, à type de porte-empreinte, et l'application à la fabrication d'un appareil buccodentaire d'immobilisation en protrusion de l'arc mandibulaire ainsi qu'un tel appareil destiné à améliorer de la ventilation pulmonaire d'un sujet et essentiellement la ventilation nocturne.

### Arrière-plan technologique

Les difficultés ou gènes respiratoires nocturnes, notamment les ronflements ou l'apnée du sommeil, sont des problèmes fréquents. De nombreuses techniques sont proposées afin de les supprimer ou les réduire. Pour les plus graves, des systèmes à masques avec ventilation assistée sont mis en oeuvre et cela représente un coût non négligeable en frais de santé. Pour les ronflements, il est proposé des dispositifs buccaux articulés qui sont en pratique difficilement supportables car peu anatomiques.

La présente invention propose un dispositif de réglage, mesure et enregistrement des rapports d'occlusion en protrusion mandibulaire particulier permettant de fabriquer un appareil buccodentaire qui est destiné à faciliter la ventilation. L'appareil en question qui peut être fabriqué, est anatomique en ce sens qu'il est adapté à la forme des arcs dentaires du sujet le portant et qu'il est maintenu en place du fait de cette adaptation.

On connait déjà des dispositifs pour prise d'empreinte ou permettant une adaptation à la dentition d'un sujet et par exemple ceux des documents GB2537313, US5313960 et US20030234022. On connait également des appareils buccodentaires destinés à remédier à certains problèmes et par exemple ceux des documents EP2491901B1, US5829441A, EP1659998B1 et EP1838259B1, mais ils sont relativement complexes et encombrants. On connait également le document US 5 868 138 A.

### Exposé de l'invention

On propose selon l'invention, un dispositif de réglage des rapports d'occlusion en protrusion mandibulaire à moyens de réglage du positionnement en protrusion de la mandibule par rapport au maxillaire.

Selon l'invention, le dispositif de réglage comporte :
- une coque allongée en arc de section en forme approximative de U inversé avec une face interne et une face externe, ladite coque ayant une portion antérieure vers l'avant et deux portions latérales droite et gauche vers l'arrière, ladite coque étant adaptée à être placée par sa face interne en recouvrement sur un arc dentaire mandibulaire d'un sujet pour prise d'empreinte dudit arc dentaire mandibulaire, une partie supérieure de la face externe venant en regard de faces occlusales de dents de l'arc dentaire maxillaire,
- au moins un dispositif d'appui réglable de réglage de protrusion, ledit au moins un dispositif d'appui comportant une pièce d'appui solidaire de la portion antérieure de la coque et destinée à venir en appui contre des dents d'un arc dentaire maxillaire, la pièce d'appui pouvant être avancée ou reculée par rapport à la coque pour réglage de la protrusion.

D'autres caractéristiques non limitatives et avantageuses du dispositif de réglage conforme à l'invention, prises individuellement ou selon toutes les combinaisons techniquement possibles, sont les suivantes :
- la pièce d'appui est destinée à venir en appui contre des incisives de l'arc dentaire maxillaire,
- la coque est adaptée à être placée par sa face interne en recouvrement avec jeu sur l'arc dentaire mandibulaire pour permettre la présence d'une pâte de prise d'empreinte dans la coque, côté face interne de la coque,
- les moyens de réglage du positionnement en protrusion permettent en outre d'enregistrer le réglage en protrusion et la pièce d'appui est solidaire de la portion antérieure de la coque par l'intermédiaire une tige filetée immobilisée à la coque, ladite pièce d'appui pouvant coulisser le long de ladite tige filetée et étant prise en sandwich entre un ressort côté coque et un écrou côté extrémité libre de la tige filetée, le ressort poussant la pièce d'appui contre l'écrou, ledit écrou pouvant être vissé ou dévissé de la tige filetée pour avancer ou reculer la pièce d'appui par rapport à la coque,
- le dispositif de réglage à moyens de réglage du positionnement en protrusion permettant en outre d'enregistrer le réglage en protrusion peut être qualifié de dispositif de réglage et d'enregistrement,
- la pièce d'appui peut en outre basculer le long de la tige filetée,
- la pièce d'appui est destinée à venir en appui contre la face avant des dents de l'arc dentaire maxillaire,
- la pièce d'appui est en forme de Y avec deux branches divergentes et un tronc commun, ledit tronc commun de la pièce d'appui étant solidaire de la portion antérieure de la coque, une des branches du Y étant destinée à venir en appui contre la face avant des dents de l'arc dentaire maxillaire et l'autre branche étant destinée à passer sous la pointe des dents de l'arc dentaire maxillaire,
- le tronc commun de la pièce d'appui est solidaire de la portion antérieure de la coque par l'intermédiaire de la tige filetée,
- le dispositif de réglage est en outre à moyens de réglage de la surélévation de l'occlusion (= réglage de l'ouverture de la mandibule par rapport au maxillaire), le dispositif de réglage comportant en outre au moins un dispositif d'écartement réglable de réglage de l'ouverture, ledit au moins un dispositif d'écartement étant solidaire de la partie supérieure de la face externe d'une portion latérale de la coque et comportant une pièce de soutien destinée à venir contre des faces occlusales de dents d'un arc dentaire maxillaire, la pièce de soutien pouvant être montée ou descendue pour réglage de l'ouverture, de préférence le dispositif de réglage comportant deux dispositifs d'écartement réglable de réglage de l'ouverture, un sur chaque portion latérale de la coque,
- les moyens de réglage de la surélévation de l'occlusion permettent en outre d'enregistrer le réglage de la surélévation de l'occlusion et le dispositif d'écartement réglable comporte une base fixée sur la partie supérieure de la face externe la de portion latérale de la coque, ladite base formant un plan incliné sur lequel la pièce de soutien en forme de coin peut coulisser pour monter ou descendre en fonction du sens de rotation d'une vis,
- la coque comporte au moins un passage à travers la portion antérieure de la coque pour contrôle de réglage de protrusion et destiné à permettre la visualisation d'au moins une des incisives de l'arc dentaire mandibulaire, la coque comportant en outre à sa face externe vers le haut de la coque et en bordure dudit passage une échelle graduée allongée antéropostérieurement destinée à mesurer l'avancement en protrusion entre les incisives de de l'arc dentaire mandibulaire et de de l'arc dentaire maxillaire,
- l'échelle graduée peut basculer entre une position où elle est allongée antéropostérieurement pour permettre la mesure de l'avancement en protrusion entre les incisives de de l'arc dentaire mandibulaire et de de l'arc dentaire maxillaire et une position où elle est allongée verticalement pour permettre la mesure de l'ouverture de la surélévation de l'occlusion entre les incisives de de l'arc dentaire mandibulaire et de de l'arc dentaire maxillaire,
- le dispositif de réglage à moyens de mesure peut être qualifié de dispositif de réglage et de mesure,
- le dispositif de réglage à moyens de réglage du positionnement en protrusion permettant en outre d'enregistrer le réglage en protrusion et à moyens de mesure peut être qualifié de dispositif de réglage, mesure et enregistrement,
- le dispositif de réglage peut permettre en outre des mesures et/ou des enregistrements,
- la coque comporte au moins un passage pour garnissage permettant l'introduction à l'intérieur de la coque, côté face interne de la coque, d'une pâte de prise d'empreinte,
- le passage pour contrôle de réglage constitue également un passage pour garnissage et inversement,
- la face interne de la coque comporte une structuration de surface afin d'assurer une meilleure rétention de la pâte de prise d'empreinte dans la coque, la structuration étant choisie parmi des indentations, des trous borgnes...
- la coque comporte des trous traversants destinés à assurer une meilleure rétention de la pâte de prise d'empreinte dans la coque,
- la coque est dans une matière transparente ou translucide.

L'invention propose également un procédé de fabrication d'un appareil buccodentaire d'immobilisation en protrusion de l'arc mandibulaire et en surélévation de l'occlusion pour un sujet, ledit appareil pouvant comporter une ou plusieurs des caractéristiques décrites et dans lequel procédé :
- dans un premier temps, on réalise sur le sujet, d'une part, une empreinte de l'arc dentaire maxillaire et, d'autre part, une empreinte de l'arc dentaire mandibulaire, la prise de l'empreinte de l'arc dentaire mandibulaire étant effectuée avec un dispositif de réglage à moyens de réglage du positionnement en protrusion et de réglage en ouverture de la mandibule par rapport au maxillaire et permettant en outre d'enregistrer le réglage en protrusion et en ouverture et lors de la prise de l'empreinte de l'arc dentaire mandibulaire, on règle et enregistre la protrusion et l'ouverture pour le sujet,
- dans un deuxième temps on réalise un modèle de l'arc dentaire maxillaire et un modèle de l'arc dentaire mandibulaire à partir des empreintes des arcs dentaires maxillaire et mandibulaire, on installe les deux modèles dans un porte-modèle ajustable et on ajuste ledit porte-modèle en plaçant sur le modèle de l'arc dentaire mandibulaire, le dispositif de réglage ayant enregistré le réglage en protrusion et ouverture afin de configurer le porte-modèle avec le même réglage en protrusion et ouverture que sur le sujet,
- dans un troisième temps, le dispositif de réglage ayant été retiré du porte-modèle, on réalise l'appareil sur le modèle de l'arc dentaire mandibulaire installé dans le porte-modèle configuré.

D'autres caractéristiques non limitatives et avantageuses du procédé conforme à l'invention, prises individuellement ou selon toutes les combinaisons techniquement possibles, sont les suivantes :
- les empreintes sur le sujet sont réalisées avec des alginates,
- après réalisation de l'appareil sur le modèle de l'arc dentaire mandibulaire installé dans le porte-modèle configuré, on retire du porte-modèle l'appareil et on le fourni au sujet ou on s'en sert pour le moulage d'un appareil qui sera fourni au sujet,
- le dispositif de réglage à moyens de réglage du positionnement en protrusion et de réglage en ouverture de la mandibule par rapport au maxillaire comporte des moyens de mesure des valeurs de protrusion et d'ouverture,
- les moyens de mesure des valeurs de protrusion et d'ouverture sont des graduations en unités de longueur,
- l'enregistrement des réglages en protrusion et en ouverture est effectué sur le papier ou un moyen de mémorisation informatique, les valeurs de protrusion et d'ouverture mesurées du réglage y étant notées ou saisies,
- l'enregistrement des réglages en protrusion et en ouverture est effectué sur le dispositif de réglage lui-même qui reste dans la position réglée obtenue sur le sujet,
- la réalisation de l'appareil consiste à appliquer de la matière sur le modèle de l'arc dentaire mandibulaire installé dans le porte-modèle configuré afin de former une gouttière renversée recouvrant les dents de l'arc mandibulaire et dont la face supérieure de l'appareil vient au contact d'au moins une partie faces occlusales des molaires droites et gauches du modèle de l'arc dentaire maxillaire par application d'une épaisseur déterminée de matière entre le fond de gouttière et ladite face supérieure, et, en outre, on applique de la matière à ladite face supérieure de l'appareil afin de former deux ailettes érigées sur ladite face supérieure et étendues vers le haut, chaque ailette venant en appui contre des faces vestibulaire (= latérales externes, côté des joues) de dents du modèle de l'arc dentaire maxillaire, et de préférence contre les dents respectivement 13 à 14 et 23 à 24,
- dans une variante plus simple, le dispositif de réglage ne comporte qu'un moyen de réglage positionnement en protrusion de la mandibule par rapport au maxillaire et l'appareil ne permet que l'immobilisation en protrusion,
- dans une variante plus simple, le dispositif de réglage ne comporte qu'un moyen de réglage en ouverture de la mandibule par rapport au maxillaire et l'appareil ne permet que l'immobilisation en surélévation de l'occlusion.

Il est également proposé l'application du dispositif de réglage dans le procédé de fabrication tel que décrit.

L'invention concerne également un appareil buccodentaire d'immobilisation en protrusion de l'arc mandibulaire par rapport à l'arc maxillaire, ledit appareil en forme de gouttière renversée étant destiné à venir recouvrir les dents de l'arc mandibulaire, ladite gouttière renversée étant ouverte vers le bas, fermée sur les côtés par des parois latérale interne côté intérieur de la bouche et latérale externe côté des joues et fermée en haut par un fond de gouttière, ledit appareil comportant trois parties principales, une partie antérieure vers l'avant destinée à venir recouvrir des incisives et canines de la mandibule et deux parties latérales droite et gauche vers l'arrière destinées à venir recouvrir les prémolaires et molaires du côté correspondant de la mandibule, la forme intérieure de la gouttière étant ajustée aux dents de la mandibule et correspondant à une empreinte desdites dents de la mandibule, l'appareil comportant une face supérieure, ladite face supérieure étant destinée à venir approximativement en regard des faces occlusales des dents du maxillaire.

Selon l'invention, l'appareil est fabriqué selon le procédé de l'invention et il comporte deux ailettes érigées sur la face supérieure et étendues vers le haut, chaque ailette étant positionnée approximativement dans la zone de raccordement entre la partie antérieure et la partie latérale droite ou gauche correspondante, et s'étendant le long de l'arc mandibulaire sur une largeur déterminée, les ailettes comportant une face intérieure orientée vers l'intérieur de la bouche et destinée à venir en appui contre des faces vestibulaire (= latérales externes, côté des joues) de dents du maxillaire lorsque l'appareil est en place en bouche afin de bloquer en protrusion la mandibule (empêche la mandibule de reculer).

D'autres caractéristiques non limitatives et avantageuses de l'appareil conforme à l'invention, prises individuellement ou selon toutes les combinaisons techniquement possibles, sont les suivantes :
- les faces occlusales des incisives du maxillaire ne viennent pas au contact de la face supérieure de l'appareil,
- chaque ailette s'étend le long de l'arc mandibulaire sur une largeur déterminée inférieure à environ 15 mm,
- avantageusement, chaque ailette s'étend le long de l'arc mandibulaire sur une largeur déterminée inférieure à environ 12 mm,
- chaque ailette s'étend le long de l'arc mandibulaire sur une largeur qui n'est pas inférieure à environ 5 mm,
- l'appareil permet en outre l'immobilisation en surélévation de l'occlusion, au moins une partie des molaires droites et gauches du maxillaire s'appuyant par leurs faces occlusales sur la face supérieure de l'appareil lorsque l'appareil buccodentaire est en place en bouche, l'appareil comportant une épaisseur déterminée de matière entre le fond de gouttière et la face supérieure au moins dans les zones où ladite au moins une partie des molaires droites et gauches du maxillaire s'appuient sur la face supérieure de l'appareil afin de bloquer la fermeture et laisser un écart de hauteur déterminé entre les incisives de la mandibule et du maxillaire,
- la paroi latérale externe de l'appareil comporte des surépaisseurs de matière permettant d'écarter les parties vestibulaires des joues à distance des arcades dentaires,
- chaque ailette a une largeur déterminée égale au maximum à une dent déterminée du maxillaire,
- la dent déterminée du maxillaire est la canine et la prémolaire adjacente,
- la dent déterminée du maxillaire est la prémolaire,
- chaque ailette est conformée selon la forme de la canine ou de la prémolaire,
- chaque ailette a une largeur déterminée égale au maximum à deux dents déterminées du maxillaire,
- les deux dents déterminées du maxillaire sont la canine et la prémolaire adjacente,
- chaque ailette est conformée selon la forme de la canine et de la prémolaire adjacente,
- chaque ailette a une largeur déterminée égale au maximum à deux dents déterminées du maxillaire que sont la canine et la prémolaire adjacente et est conformée pour s'appliquer à cheval entre la canine et la prémolaire adjacente,
- l'appareil est configuré afin que les faces intérieures des deux ailettes viennent s'appliquer sur les faces vestibulaires des dents respectivement 13 à 14 et 23 à 24 du maxillaire,
- l'appareil est configuré pour immobiliser l'arc mandibulaire par rapport à l'arc maxillaire en protrusion d'une valeur d'avancement déterminée, ladite valeur d'avancement étant dans la gamme de 1 mm à 4 mm,
- la valeur d'avancement est de préférence inférieure ou égale à la moitié de la capacité de protrusion maximale du sujet,
- la valeur d'avancement est mesurée bout à bout des incisives centrales des arcs dentaires maxillaire et mandibulaire,
- la valeur d'avancement est mesurée bout à bout des incisives centrales des arcs dentaires maxillaire et mandibulaire, sur une horizontale,
- la valeur d'avancement est avantageusement dans la gamme de 1 mm à 3 mm,
- l'appareil est configuré pour immobiliser l'arc mandibulaire par rapport à l'arc maxillaire en surélévation de l'occlusion d'une valeur d'ouverture déterminée, ladite valeur d'ouverture étant dans la gamme de 1 mm à 10 mm,
- la valeur d'ouverture est avantageusement dans la gamme de 1 mm à 9 mm,
- la valeur d'ouverture est plus avantageusement dans la gamme de 3 mm à 9 mm,
- la valeur d'ouverture est mesurée bout à bout des incisives centrales des arcs dentaires maxillaire et mandibulaire,
- la valeur d'ouverture est mesurée bout à bout des incisives centrales des arcs dentaires maxillaire et mandibulaire, sur une verticale,
- l'appareil est configuré pour écarter les parties vestibulaires des joues à distance des arcades dentaires d'une valeur d'écartement déterminée, ladite valeur d'écartement étant dans la gamme de 4 mm à 10 mm,
- l'appareil est dans une matière qui est une matière plastique choisie parmi les poly-acétates dentaires, la FLEXITE^{®}, le VALPLAST^{®}, les résines acryliques dentaires, les matières plastiques thermoformables dentaires, l'ERKOFLEXO,
- la matière est sensiblement transparente ou translucide,
- la matière est colorée dans la masse,
- la partie antérieure de l'appareil est amincie,
- la partie antérieure amincie de l'appareil comporte un siège de réception d'un accessoire,
- l'accessoire est amovible,
- l'accessoire est un insert de communication aérique interlabiale entre l'intérieur de la cavité buccale et l'environnement extérieur,
- l'insert comporte une assise en U destinée à venir se positionner à cheval sur le siège de la partie antérieure de l'appareil,
- l'assise et le siège comportent des moyens complémentaires assurant une retenue de l'insert sur l'appareil,
- l'insert comporte au moins un tube creux destiné à faire communiquer aériquement l'intérieur de la cavité buccale et l'environnement extérieur,
- l'insert comporte un tube creux médian,
- l'insert comporte deux tubes creux latéraux.

### Brève description des dessins

**[****Fig. 1****]** représente une vue frontale/avant plongeante d'un appareil buccodentaire fabriqué selon l'invention,
**[****Fig. 2****]** représente une vue latérale droite plongeante de l'appareil buccodentaire de la figure 1,
**[****Fig. 3****]** représente une vue latérale gauche plongeante de l'appareil buccodentaire de la figure 1,
**[****Fig. 4****]** représente une vue arrière plongeante de l'appareil buccodentaire de la figure 1,
**[****Fig. 5****]** représente une vue de dessous de l'appareil buccodentaire de la figure 1,
**[****Fig. 6****]** représente une vue latérale d'un porte-modèle ajustable dans lequel deux modèles des arcs dentaires mandibulaire et maxillaire ont été installés ainsi que l'appareil dentaire réalisé sur le modèle de l'arc dentaire mandibulaire,
**[****Fig. 7****]** représente une vue d'un premier côté, en perspective, d'un dispositif de réglage selon l'invention à moyens de réglage du positionnement en protrusion et de réglage en ouverture, de la mandibule par rapport au maxillaire,
**[****Fig. 8****]** représente une vue d'un second côté, en perspective, du dispositif de réglage de la figure 7,
**[****Fig. 9****]** représente une vue de dessus du dispositif de réglage de la figure 7,
**[****Fig. 10****]** représente une première vue en perspective d'un insert amovible de communication aérique interlabiale entre l'intérieur de la cavité buccale et l'environnement extérieur et qui peut être installé sur la partie antérieure amincie de l'appareil, et
**[****Fig. 11****]** représente une seconde vue en perspective de l'insert de la figure 10.

### Description détaillée d'un exemple de réalisation

La description qui va suivre en regard des dessins annexés, donnés à titre d'exemples non limitatifs, fera bien comprendre en quoi consiste l'invention et comment elle peut être réalisée.

Le dispositif de réglage de l'invention est destiné à réaliser une prise d'empreinte de l'arc dentaire mandibulaire dans des conditions particulières de protrusion et de surélévation de l'ouverture de la mandibule par rapport au maxillaire sur un sujet. De préférence, le dispositif de réglage peut enregistrer la protrusion et l'ouverture pour recréer directement sur un modèle buccal les mêmes conditions de protrusion et d'ouverture que sur le sujet, l'appareil buccodentaire étant réalisé sur le modèle buccal. Le modèle buccal est constitué d'un porte-modèle avec les modèles des arcs dentaires mandibulaire et maxillaire obtenus par prises d'empreintes sur le sujet.

L'appareil buccodentaire fabriqué grâce au dispositif de réglage de l'invention est simple, original et léger. En particulier, il ne comporte pas d'articulation et est en relation avec un minimum de structures anatomiques du sujet. En particulier seulement deux petites ailettes s'appuyant chacune sur les faces vestibulaires de seulement deux dents adjacentes au maximum du maxillaire permettent de bloquer en protrusion la mandibule. L'appareil buccodentaire agit par trois actions principales afin d'améliorer la ventilation, en particulier la ventilation nocturne, et/ou diminuer, voire supprimer, les ronflements.

Ces actions sont :
1°) la protrusion mandibulaire,
2°) la surélévation de l'occlusion,
3°) l'amélioration de la tonicité tissulaire au niveau des joues et des lèvres par le gonflement des deux parties vestibulaires du sujet.

Si la combinaison de ces actions permet d'obtenir des résultats particulièrement intéressants, chacune de ces actions ou des combinaisons de deux d'entre elles permettent également d'obtenir des résultats utiles.

En ce qui concerne la protrusion mandibulaire permise par l'appareil de la présente invention, on doit noter qu'elle est obtenue d'une manière bien plus simple et élégante que dans les appareillages existants où les gouttières haute et basse sont reliées par des charnières qui forcent la mandibule à se fermer dans une position déterminée. Ces appareillages existants ne sont pas très confortables pour le sujet à cause de douleurs provoquées au niveau des articulations temporo mandibulaires, entrainant des réveils plusieurs fois la nuit et provoquant un encombrement des voies aériennes.

Pour rappel du contexte, l'arcade dentaire supérieure, dite maxillaire, surplombe l'arcade dentaire inferieure, dite mandibule. L'arcade supérieure ou maxillaire faisant partie de l'ensemble cranio facial est fixe et l'arcade inferieure ou mandibule est articulée et mobile.

On va commencer par décrire l'appareil buccodentaire qui est fabriqué en utilisant le dispositif de réglage de l'invention avant de décrire le procédé de fabrication puis décrire plus précisément le dispositif de réglage utilisé.

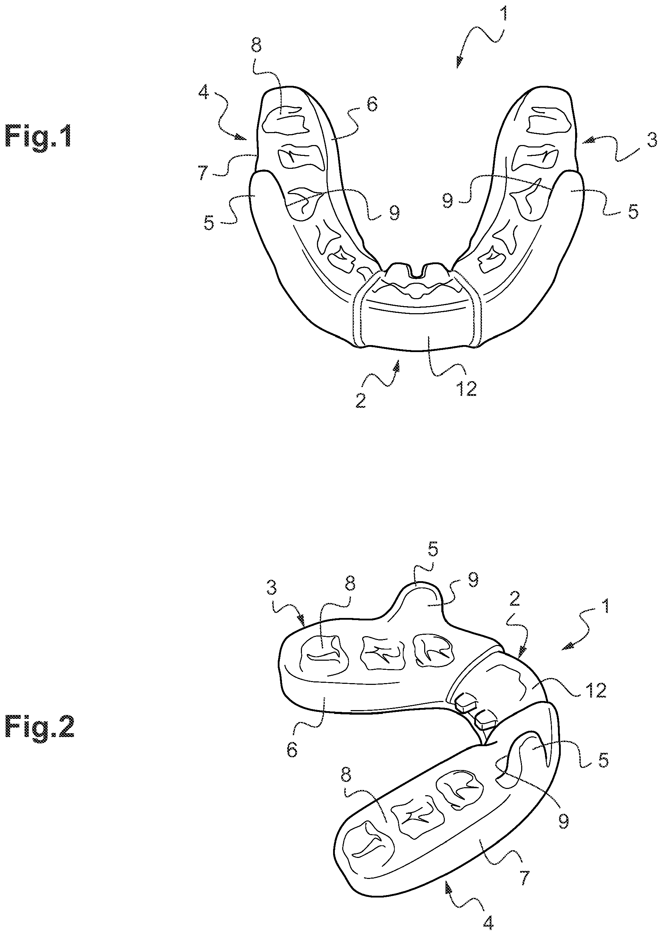

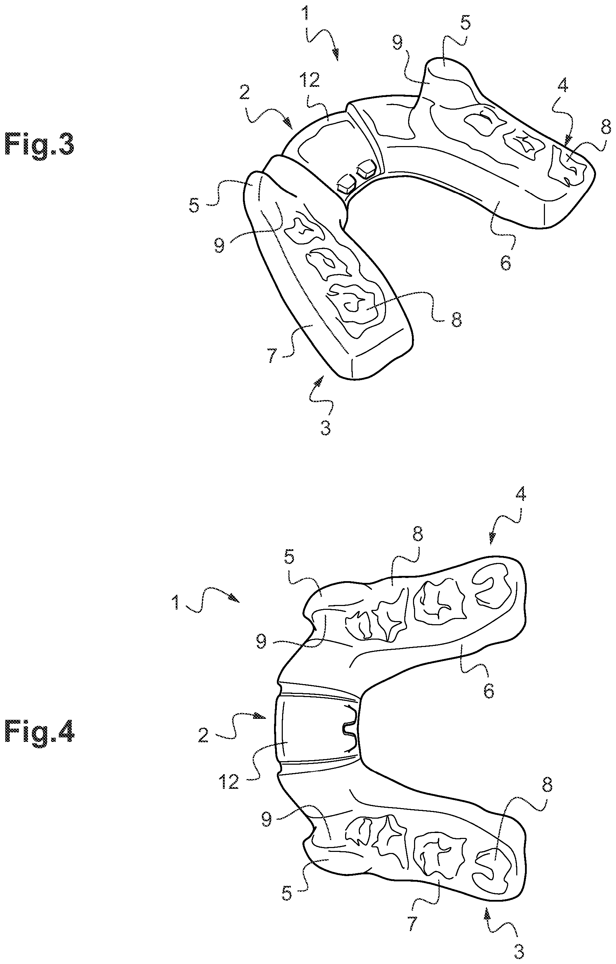

L'appareil buccodentaire 1 des figures 1 à 5 permet une immobilisation de la mandibule en protrusion avec une valeur d'avancement déterminée de la mandibule par rapport au maxillaire. L'appareil buccodentaire 1 permet aussi de maintenir la mandibule en surélévation de l'occlusion, c'est-à-dire en ouverture de la cavité buccale, avec une valeur d'ouverture déterminée. Cet appareil 1 présente trois parties principales, une partie antérieure 2 vers l'avant et qui est destinée à venir recouvrir des incisives et canines de la mandibule et deux parties latérales droite 4 et gauche 3 vers l'arrière destinées à venir recouvrir les prémolaires et molaires du côté correspondant de la mandibule. Les étendues respectives de ces trois parties sont indicatives et sont données pour faciliter les explications. En pratique, la zone de transition entre la partie antérieure 2 et chaque partie latérale 3, 4 peut se situer entre les canines et prémolaires ou, moins préférentiellement, entre les prémolaires ou, encore, entre les prémolaires et molaires.

L'appareil 1 présente une forme de gouttière 10 renversée qui épouse la forme de l'arc dentaire mandibulaire et la forme de ses dents car il a été réalisé sur un modèle moulé de l'arc dentaire mandibulaire d'un sujet auquel l'appareil est destiné. Cette gouttière comporte deux parois latérales, une paroi latérale interne 6 côté intérieur de la bouche et de la langue, et une paroi latérale externe 7 (= vestibulaire) côté des joues. Cette gouttière 10 comporte un fond 11 de gouttière qui la ferme vers le haut. Ce fond 11 de gouttière constitué d'une épaisseur de matière forme, côté supérieur de ladite matière, une face supérieure 8 destinée à venir approximativement en regard des faces occlusales des dents du maxillaire. L'épaisseur de matière entre le fond 11 de la gouttière 10 et la face supérieure 8 dans les parties latérales 3, 4 de l'appareil 1 détermine l'importance/la grandeur de surélévation de l'occlusion. Plus cette épaisseur sera grande, plus la surélévation de l'occlusion et donc la valeur d'ouverture sera grande.

L'intérieur de la gouttière 10 et en particulier son fond 11 comportent donc des empreintes des dents de la mandibule. De même, la face supérieure 8 de l'appareil 1 comporte des empreintes de faces occlusales de dents du maxillaire et plus particulièrement des dents arrière, essentiellement molaires. On doit noter qu'à l'avant de l'appareil on ne trouve pas d'empreinte de dents du maxillaire du fait d'une part de la protrusion et, surtout, du blocage en surélévation de l'occlusion. On comprend que ces empreintes sur la face supérieure 8 de l'appareil 1 seront plus ou moins étendues/présentes en allant vers l'avant que la surélévation de l'occlusion est moins ou plus grande.

L'appareil 1 comporte deux ailettes 5 érigées sur la face supérieure 8 approximativement dans la zone entre la partie antérieure 2 et les parties latérales 3, 4. Chacune de ces ailettes 5 s'étend le long de l'arc mandibulaire sur une largeur déterminée très réduite, inférieure à environ 12 mm, et correspondant en pratique et au maximum à l'étendue de deux dents du maxillaire sur lesquelles l'ailette vient s'appliquer. Ces deux dents du maxillaire sont de préférence 13, 14 et 23, 24 selon la nomenclature classiquement utilisée et qui correspondent à des canines et des premières prémolaires. D'ailleurs, on peut voir sur les figures de l'appareil 1 que la face intérieure 9 des ailettes 5 comporte des empreintes des dents du maxillaire sur lesquelles ces ailettes 5 s'appliquent. De préférence et comme représenté, les ailettes 5 ont une base plus large qu'à leur sommet. Du fait de la forme en arc de la mandibule et du maxillaire et des formes des dents, notamment d'un retrait existant entre deux dents, ces ailettes 5 pourtant de largeur réduite, permettent un maintien efficace de la mandibule en protrusion et, ceci, avec un minimum de gène et d'encombrement en bouche.

De préférence, la partie antérieure 2 de l'appareil 1 comporte une épaisseur de matière plus réduite que le reste de l'appareil afin de ne pas trop réduire la surface de passage de l'air entre les dents avant du maxillaire et la partie antérieure 2 de l'appareil 1, afin que le sujet porteur de l'appareil puisse continuer à respirer par la bouche convenablement.

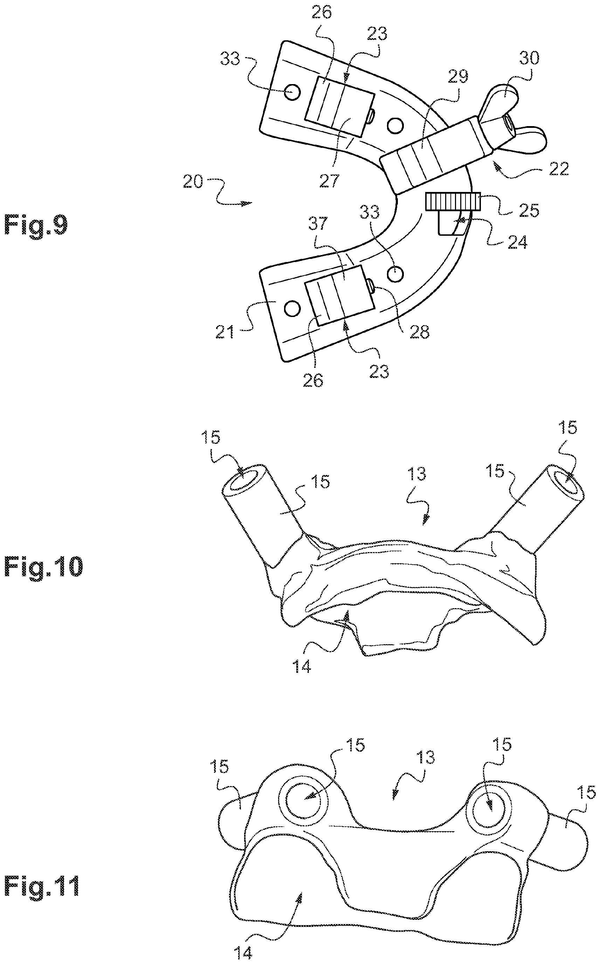

Cette épaisseur de matière plus réduite de la partie antérieure 2 peut aussi correspondre à un siège 12 ou comporter un siège 12 de réception d'un accessoire amovible que l'on peut installer sur l'appareil 1 en cas de besoin et retirer si on n'en a plus besoin. Cet accessoire peut être un insert 13 de communication aérique interlabiale entre l'intérieur de la cavité buccale et l'environnement extérieur de l'utilisateur de l'appareil et dont un exemple est représenté sur les figures 10 et 11. Cet insert 13 comporte une assise 14 en U destinée à venir se positionner à cheval sur le siège 12 de l'appareil. L'assise 14 et le siège 12 peuvent comporter des moyens complémentaires assurant une certaine retenue de l'insert 13 sur l'appareil 1. Ces moyens complémentaires sont par exemple des protubérances de l'assise de l'insert rentrant d'une manière résiliente dans des creux complémentaires du siège 12. L'assise peut déborder latéralement du siège et donc comporter des parties moins épaisses latéralement.

L'insert 13 de communication aérique interlabiale comporte au moins un tube 15 creux destiné à faire communiquer aériquement l'intérieur de la cavité buccale et l'environnement extérieur même lorsque l'utilisateur ferme les lèvres puisque le/les tubes 15 passent entre les lèvres. Sur la figure, l'insert 13 comporte deux tubes 15 dont on peut voir les orifices. L'insert 13 installé sur l'appareil 1 permet ainsi d'assurer un certain niveau d'échange gazeux entre le système respiratoire et l'environnement, ce qui est notamment intéressant dans le cas où l'utilisateur a un système nasal rétréci, voire obstrué. Les tubes peuvent être rigides ou non, de préférence avec une certaine résistance radiale leur permettant de rester au moins en partie ouverts lorsque la personne serre les lèvres et/ou les dents.

L'invention peut aussi se présenter sous forme d'un kit utilisable par un utilisateur et qui comporte, d'une part, l'appareil 1 et, d'autre part, un ou plusieurs inserts 13 de communication aérique interlabiale, les inserts pouvant être de plusieurs types : à un ou deux ou plus tubes 15.

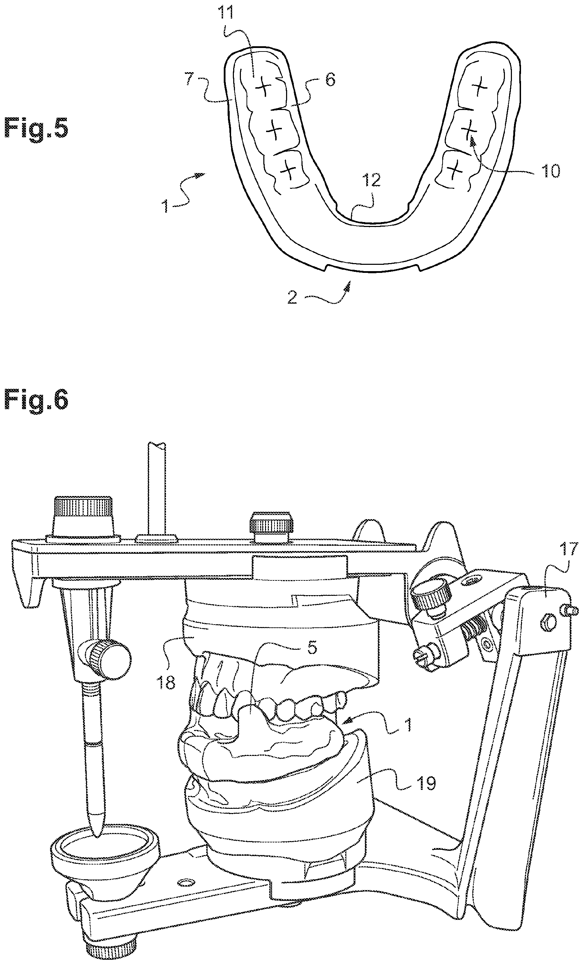

En relation avec la figure 6, on va maintenant décrire le procédé de fabrication de l'appareil buccodentaire 1. De préférence, cet appareil est fabriqué sur mesure pour un sujet donné mais, dans des variantes de réalisation, il est possible de fabriquer des appareils standardisés utilisables par des groupes d'utilisateurs ayant des caractéristiques communes et en particulier de taille et/ou dimensions anatomiques buccales. Dans des variantes plus évoluées, on propose des appareils standardisés permettant une certaine adaptation au sujet l'utilisant, par exemple permettant un moulage de l'appareil standardisé aux dents du sujet. Il existe en effet des matières thermoplastiques pouvant permettre une prise d'empreintes de dents et on prévoit d'en mettre dans la gouttière 10 élargie d'un appareil standardisé, le sujet chauffant son appareil standardisé et faisant une prise d'empreinte de sa mandibule pour conformer son appareil standardisé à son arc dentaire mandibulaire.

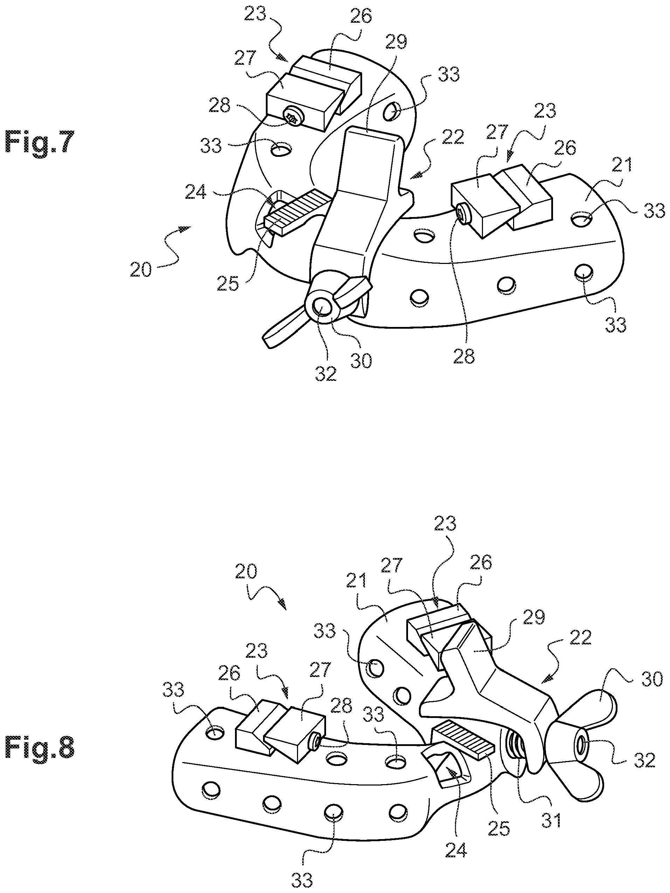

Afin d'obtenir un appareil 1 sur mesure pour un sujet donné, dans un premier temps, on réalise sur le sujet, d'une part, une empreinte de l'arc dentaire maxillaire sans contrainte particulière. D'autre part, on réalise sur le sujet une empreinte de l'arc dentaire mandibulaire mais cette fois avec la contrainte que la prise de l'empreinte de l'arc dentaire mandibulaire est effectuée avec un dispositif de réglage à moyens de réglage du positionnement en protrusion et de réglage en ouverture de la mandibule par rapport au maxillaire et qui permet en outre d'enregistrer les réglages en protrusion et en ouverture. Lors de la prise de cette empreinte de l'arc dentaire mandibulaire, on aura réglé et enregistré la protrusion et l'ouverture pour le sujet. De préférence, cet enregistrement de la protrusion et de l'ouverture se fait sur le dispositif de réglage à moyens de réglage du positionnement en protrusion et de réglage en ouverture qui reste dans la configuration réglée (ce qui correspond à un enregistrement/mémorisation par le dispositif de réglage que l'on pourrait alors qualifier de dispositif de réglage et d'enregistrement). On peut prévoir que les moyens de réglage soient escamotables mais qu'après escamotage, ils reprennent la position correspondant au réglage obtenu sur le sujet. C'est le cas pour le réglage de protrusion avec le dispositif de réglage des figures 7 à 9 où, comme on le verra, un ressort est utilisé pour le dispositif d'appui permettant le réglage en protrusion. Dans une variante, on utilise des moyens de mesure de la valeur d'avancement de la protrusion et des moyens de mesure de la valeur d'ouverture de surélévation de l'occlusion et on les note ou on les enregistre sur un support papier ou autre pour une utilisation ultérieure. De préférence, le dispositif de réglage à moyens de réglage du positionnement en protrusion et de réglage en ouverture comporte aussi de tels moyens de mesure (le dispositif de réglage pourrait alors être qualifié de dispositif de réglage et mesure ou de dispositif de réglage, mesure et enregistrement en fonction de la présence ou non de moyens d'enregistrement) permettant ainsi de l'utiliser selon différentes possibilités et notamment celles décrites à titre d'exemple.

Une fois ces empreintes maxillaire et mandibulaire obtenues et dans un deuxième temps, on réalise un modèle de l'arc dentaire maxillaire et un modèle de l'arc dentaire mandibulaire. On installe les deux modèles dans un porte-modèle ajustable et on ajuste ledit porte-modèle en plaçant sur le modèle de l'arc dentaire mandibulaire, le dispositif de réglage ayant enregistré le réglage en protrusion et ouverture afin de configurer le porte-modèle avec le même réglage en protrusion et ouverture que sur le sujet. On comprend que si le dispositif de réglage à moyens de réglage du positionnement en protrusion et de réglage en ouverture ne permet pas l'enregistrement des valeurs d'avancement et d'ouverture (ou qu'elles ont été modifiées ou perdues) et qu'on les a mesurées et enregistrées ailleurs au premier temps, on réglera le dispositif de réglage pour le reconfigurer avec lesdites valeurs dans le porte-modèle puis on configure le porte-modèle pour correspondre à ce qui avait été réglé sur le sujet.

Une fois le dispositif de réglage retiré du porte-modèle, on dispose alors d'un porte-modèle avec les modèles des arcs dentaires qui a une configuration identique en protrusion et ouverture à celle qui avait été obtenue sur le sujet au premier temps. On peut alors, dans un troisième temps, réaliser l'appareil sur ce porte-modèle configuré comme sur le sujet. Pour cela, on applique de la matière sur le modèle de l'arc dentaire mandibulaire installé dans le porte-modèle configuré afin de former une gouttière 10 renversée recouvrant les dents de l'arc mandibulaire. On s'assure que l'épaisseur de matière entre le fond 11 de gouttière et la face supérieure 8 est telle que la face supérieure 8 de l'appareil vient au contact d'au moins une partie des faces occlusales des molaires droites et gauches du modèle de l'arc dentaire maxillaire afin d'obtenir la surélévation d'occlusion attendue/prévue. En outre, on réalise les deux ailettes 5 par application de matière à la face supérieure 8 de l'appareil, chaque ailette venant en appui contre des faces vestibulaire de dents du modèle de l'arc dentaire maxillaire, et de préférence contre les dents respectivement 13 à 14 et 23 à 24.

La figure 6 permet de visualiser l'appareil 1 réalisé dans le porte-modèle 17 comportant le modèle de l'arc dentaire maxillaire 18 et le modèle de l'arc dentaire mandibulaire 19. On peut voir que le porte-modèle comporte des moyens de réglage de la protrusion et de l'ouverture qui ont été configurés au deuxième temps pour correspondre à ceux du sujet grâce à l'utilisation du dispositif de réglage qui a enregistré le réglage de protrusion et d'ouverture sur le sujet.

L'appareil 1 réalisé dans le porte-modèle peut être celui directement fourni au sujet ou, de préférence, il peut servir au moulage d'un appareil qui sera fourni au sujet. Pour la matière utilisable, tout type de résine peut être utilisé pour la réalisation de l'appareil du moment qu'elle est compatible avec la confection d'appareils dentaires ou orthodontiques : FLEXITE, VALPLAST, et tous types de poly-acétates dentaires rose gencive ou transparents, tous type de résines acryliques dentaires colorées ou transparentes, tous matériaux thermo formables type ERKOFLEX + résine transparente transparent ou colorés.

On va maintenant décrire en relation avec les figures 7 à 9, un dispositif de réglage 20 à moyens de réglage du positionnement en protrusion et de réglage en ouverture de la mandibule par rapport au maxillaire et qui permet en outre d'enregistrer les réglages en protrusion et en ouverture et de mesurer ces réglages.

Le dispositif de réglage 20 comporte une coque 21 allongée en arc et ayant une section en forme approximative de U inversé avec une face interne destinée à venir recouvrir l'arc dentaire mandibulaire et une face externe. La coque 21 comporte trois portions, une portion antérieure vers l'avant et deux portions latérales droite et gauche vers l'arrière. La face interne de la coque 21 présente un certain jeu par rapport à l'arc dentaire mandibulaire qu'elle recouvre afin qu'une pâte de prise d'empreinte puisse être introduite entre la coque 21 et que le moulage des dents de la mandibule puisse s'effectuer correctement. La pâte de prise d'empreinte peut éventuellement être introduite dans la coque 21 en place en bouche par notamment un passage 24 réalisé dans la coque 21 du dispositif de réglage 20 ou être répandue dans la coque préalablement à la mise en place du dispositif de réglage sur l'arc dentaire mandibulaire. La coque 21 comporte des trous 33 traversants destinés à assurer une meilleure rétention de la pâte de prise d'empreinte dans la coque 21. Ces trous 33 peuvent aussi, éventuellement, servir à l'introduction possiblement complémentaire de pâte de prise d'empreinte. De préférence, on répand de la pâte de prise d'empreinte dans la coque du dispositif de réglage alors non en bouche puis on l'installe en bouche pour prendre les empreintes dentaires mandibulaires. Concernant le réglage de la protrusion, le dispositif de réglage 20 comporte un dispositif d'appui 22 réglable de réglage de protrusion. Le dispositif d'appui 22 est solidaire de la face externe de la portion antérieure de la coque 21 et comporte une pièce d'appui 29 en forme de Y destinée à venir en appui contre des dents de l'arc dentaire maxillaire. Cette pièce d'appui 29 peut être avancée ou reculée par coulissement le long d'une tige filetée 32 pour réglage de la protrusion et elle est réglable par vissage dévissage d'un écrou papillon 30 sur ladite tige filetée 32 solidaire de la coque, la pièce d'appui 29 étant repoussée de la coque 21 par un ressort 31, ce qui assure l'enregistrement de l'avancement pour la protrusion même si on peut ensuite escamoter/déplacer la pièce d'appui le long de la tige filetée. On comprend qu'après le réglage de la protrusion sur le sujet par action sur l'écrou 30, on ne modifiera pas la position de l'écrou 30 pour conserver/enregistrer ledit réglage. Cette pièce d'appui 29 peut se déplacer librement avec un certain jeu le long de la tige filetée 32 en fonction de la position de l'écrou papillon 30 et, de préférence, elle peut aussi basculer voire tourner.

Toujours en relation avec la protrusion mais cette fois concernant la mesure de la valeur d'avancement, le dispositif de réglage 20 comporte une échelle graduée 25 permettant de mesurer directement la distance sensiblement sur une horizontale entre les bouts respectifs des incisives centrales des arcs dentaires maxillaire et mandibulaire. Pour repérer les bouts des incisives mandibulaires, le passage 24 peut être utilisé et/ou on peut mettre en oeuvre une coque 21 transparente. Le passage 24 peut faire office de passage pour contrôle et pour mesure de réglage(s) de protrusion et/ou en ouverture.

Concernant le réglage de la surélévation en occlusion/ouverture, le dispositif de réglage 20 comporte deux dispositifs d'écartement 23 réglables de réglage de l'ouverture, un sur chaque portion latérale de la coque 21. Chaque dispositif d'écartement 23 comporte une base 26 solidaire de la partie supérieure de la face externe de la portion latérale de la coque 21 et une pièce de soutien 27 destinée à venir contre des dents d'un arc dentaire maxillaire, plus spécifiquement contre des faces occlusales de molaires. La pièce de soutien 27 peut être montée ou descendue sur la base 26 pour réglage de l'ouverture du fait des formes en coins de la pièce de soutien 27 et de la base 26. Une vis 28 permet par vissage dévissage le réglage du dispositif d'écartement 23.

Toujours en relation avec la surélévation en occlusion mais cette fois concernant la mesure de la valeur d'ouverture, on peut mesurer avec une règle rapportée/externe la distance verticale entre les bouts respectifs des incisives centrales des arcs dentaires maxillaire et mandibulaire. Dans une variante, on peut prévoir une échelle graduée du type de celle 25 mais cette fois verticale, agencée sur le dispositif de réglage. Dans une modalité particulière, l'échelle graduée 25 peut basculer entre une position sensiblement horizontale et une position sensiblement verticale.

Afin de faciliter les réglages de protrusion et d'ouverture ainsi que les mesures, on peut prévoir que la coque soit dans une matière transparente ou translucide.

On a décrit à titre d'exemple un dispositif de réglage permettant les deux réglages : de protrusion et de la surélévation en occlusion, ainsi que l'enregistrement des réglages et au moins la mesure de la valeur d'avancement pour la protrusion. Dans d'autres modalités de mise en oeuvre, le dispositif de réglage ne permet que le réglage de la protrusion ou que le réglage de de la surélévation en occlusion. Dans certaines modalités de mise en oeuvre, le dispositif de réglage ne permet pas l'enregistrement du/des réglages. Dans certaines modalités de mise en oeuvre, le dispositif de réglage ne permet pas les mesures directes, c'est-à-dire sans avoir recours à un moyen de mesure externe. Dans ces cas, les moyens correspondants sont absents du dispositif de réglage qui se trouve ainsi simplifié.

## Revendications

1. Dispositif de réglage des rapports d'occlusion en protrusion mandibulaire (20) à moyens de réglage du positionnement en protrusion de la mandibule par rapport au maxillaire, le dispositif comportant :
- une coque (21) allongée en arc de section en forme approximative de U inversé avec une face interne et une face externe, ladite coque (21) ayant une portion antérieure vers l'avant et deux portions latérales droite et gauche vers l'arrière, ladite coque (21) étant adaptée à être placée par sa face interne en recouvrement sur un arc dentaire mandibulaire d'un sujet pour prise d'empreinte dudit arc dentaire mandibulaire, une partie supérieure de la face externe venant en regard de faces occlusales de dents de l'arc dentaire maxillaire,
- au moins un dispositif d'appui (22) réglable de réglage de protrusion, ledit au moins un dispositif d'appui (22) comportant une pièce d'appui (29) solidaire de la portion antérieure de la coque (21) et destinée à venir en appui contre des dents d'un arc dentaire maxillaire, la pièce d'appui (29) pouvant être avancée ou reculée par rapport à la coque pour réglage de la protrusion,
**caractérisé en ce que** les moyens de réglage du positionnement en protrusion permettent en outre d'enregistrer le réglage en protrusion et **en ce que** la pièce d'appui (29) est solidaire de la portion antérieure de la coque (21) par l'intermédiaire une tige filetée (32) immobilisée à la coque (21), ladite pièce d'appui (29) pouvant coulisser le long de ladite tige filetée (32) et étant prise en sandwich entre un ressort (31) côté coque et un écrou (30) côté extrémité libre de la tige filetée (32), le ressort (31) poussant la pièce d'appui (29) contre l'écrou (30), ledit écrou (30) pouvant être vissé ou dévissé de la tige filetée (32) pour avancer ou reculer la pièce d'appui (29) par rapport à la coque (21).

2. Dispositif de réglage (20) selon la revendication 1, **caractérisé en ce que** la pièce d'appui (29) est en forme de Y avec deux branches divergentes et un tronc commun, ledit tronc commun de la pièce d'appui (29) étant solidaire de la portion antérieure de la coque, une des branches du Y étant destinée à venir en appui contre la face avant des dents de l'arc dentaire maxillaire et l'autre branche étant destinée à passer sous la pointe des dents de l'arc dentaire maxillaire.

3. Dispositif de réglage (20) selon l'une quelconque des revendications 1 à 2, **caractérisé en ce qu'**il est en outre à moyens de réglage de la surélévation de l'occlusion, le dispositif de réglage comportant en outre :
- au moins un dispositif d'écartement (23) réglable de réglage de l'ouverture, ledit au moins un dispositif d'écartement étant solidaire de la partie supérieure de la face externe d'une portion latérale de la coque (21) et comportant une pièce de soutien (27) destinée à venir contre des faces occlusales de dents d'un arc dentaire maxillaire, la pièce de soutien (27) pouvant être montée ou descendue pour réglage de l'ouverture, de préférence le dispositif de réglage (20) comportant deux dispositifs d'écartement (23) réglable de réglage de l'ouverture, un sur chaque portion latérale de la coque (21).

4. Dispositif de réglage (20) selon la revendication 3, **caractérisé en ce que** les moyens de réglage de la surélévation de l'occlusion permettent en outre d'enregistrer le réglage de la surélévation de l'occlusion et **en ce que** le dispositif d'écartement (23) réglable comporte une base (26) fixée sur la partie supérieure de la face externe de la portion latérale de la coque (21), ladite base (26) formant un plan incliné sur lequel la pièce de soutien (27) en forme de coin peut coulisser pour monter ou descendre en fonction du sens de rotation d'une vis (28).

5. Dispositif de réglage (20) selon l'une quelconque des revendications 1 à 4, **caractérisé en ce que** la coque (21) comporte au moins un passage (24) à travers la portion antérieure de la coque pour contrôle de réglage de protrusion et destiné à permettre la visualisation d'au moins une des incisives de l'arc dentaire mandibulaire, la coque (21) comportant en outre à sa face externe vers le haut de la coque (21) et en bordure dudit passage (24) une échelle graduée (25) allongée antéropostérieurement destinée à mesurer l'avancement en protrusion entre les incisives de de l'arc dentaire mandibulaire et de de l'arc dentaire maxillaire.

6. Dispositif de réglage (20) selon la revendication 5, **caractérisé en ce que** l'échelle graduée (25) peut basculer entre une position où elle est allongée antéropostérieurement pour permettre la mesure de l'avancement en protrusion entre les incisives de de l'arc dentaire mandibulaire et de de l'arc dentaire maxillaire et une position où elle est allongée verticalement pour permettre la mesure de l'ouverture de la surélévation de l'occlusion entre les incisives de de l'arc dentaire mandibulaire et de de l'arc dentaire maxillaire.

7. Dispositif de réglage (20) selon l'une quelconque des revendications 1 à 6, **caractérisé en ce que** la coque comporte au moins un passage (24) pour garnissage permettant l'introduction à l'intérieur de la coque, côté face interne de la coque, d'une pâte de prise d'empreinte.

8. Procédé de fabrication d'un appareil (1) buccodentaire d'immobilisation en protrusion de l'arc mandibulaire et en surélévation de l'occlusion pour un sujet, **caractérisé en ce que** :
- dans un premier temps, on réalise sur le sujet, d'une part, une empreinte de l'arc dentaire maxillaire et, d'autre part, une empreinte de l'arc dentaire mandibulaire, la prise de l'empreinte de l'arc dentaire mandibulaire étant effectuée avec un dispositif de réglage (20) à moyens de réglage du positionnement en protrusion (22) et de réglage en ouverture (23) de la mandibule par rapport au maxillaire et permettant en outre d'enregistrer le réglage en protrusion et en ouverture, ledit dispositif de réglage (20) étant selon la revendication 5 dans sa combinaison à la revendication 2, et lors de la prise de l'empreinte de l'arc dentaire mandibulaire, on règle et enregistre la protrusion et l'ouverture pour le sujet,
- dans un deuxième temps on réalise un modèle de l'arc dentaire maxillaire et un modèle de l'arc dentaire mandibulaire à partir des empreintes des arcs dentaires maxillaire et mandibulaire, on installe les deux modèles dans un porte-modèle (17) ajustable et on ajuste ledit porte-modèle (17) en plaçant sur le modèle de l'arc dentaire mandibulaire, le dispositif de réglage (20) ayant enregistré le réglage en protrusion et ouverture afin de configurer le porte-modèle (17) avec le même réglage en protrusion et ouverture que sur le sujet,
- dans un troisième temps, le dispositif de réglage (20) ayant été retiré du porte-modèle (17), on réalise l'appareil sur le modèle de l'arc dentaire mandibulaire installé dans le porte-modèle configuré, la réalisation de l'appareil (1) consistant à appliquer de la matière sur le modèle de l'arc dentaire mandibulaire installé dans le porte-modèle configuré afin de former une gouttière (10) renversée recouvrant les dents de l'arc mandibulaire et dont la face supérieure (8) de l'appareil (1) vient au contact d'au moins une partie faces occlusales des molaires droites et gauches du modèle de l'arc dentaire maxillaire par application d'une épaisseur déterminée de matière entre le fond (11) de gouttière et ladite face supérieure (8), et **en ce qu'**en outre on applique de la matière à ladite face supérieure de l'appareil afin de former deux ailettes (5) érigées sur ladite face supérieure et étendues vers le haut, chaque ailette (5) venant en appui contre des faces vestibulaire de dents du modèle de l'arc dentaire maxillaire, et de préférence contre les dents respectivement 13 à 14 et 23 à 24.

9. Appareil (1) buccodentaire d'immobilisation en protrusion de l'arc mandibulaire par rapport à l'arc maxillaire fabriqué selon le procédé de la revendication 8, ledit appareil en forme de gouttière (10) renversée étant destiné à venir recouvrir les dents de l'arc mandibulaire, ladite gouttière (10) renversée étant ouverte vers le bas, fermée sur les côtés par des parois latérale interne (6) côté intérieur de la bouche et latérale externe (7) côté des joues et fermée en haut par un fond (11) de gouttière, ledit appareil (1) comportant trois parties principales, une partie antérieure (2) vers l'avant destinée à venir recouvrir des incisives et canines de la mandibule et deux parties latérales droite (4) et gauche (3) vers l'arrière destinées à venir recouvrir les prémolaires et molaires du côté correspondant de la mandibule, la forme intérieure de la gouttière (10) étant ajustée aux dents de la mandibule et correspondant à une empreinte desdites dents de la mandibule, l'appareil comportant une face supérieure (8), ladite face supérieure étant destinée à venir approximativement en regard des faces occlusales des dents du maxillaire,
**caractérisé en ce que** l'appareil (1) comporte deux ailettes (5) érigées sur la face supérieure (8) et étendues vers le haut, chaque ailette (5) étant positionnée approximativement dans la zone de raccordement entre la partie antérieure (2) et la partie latérale droite (4) ou gauche (3) correspondante, et s'étendant le long de l'arc mandibulaire sur une largeur déterminée, les ailettes (5) comportant une face intérieure (9) orientée vers l'intérieur de la bouche et destinée à venir en appui contre des faces vestibulaire de dents du maxillaire lorsque l'appareil (1) est en place en bouche afin de bloquer en protrusion la mandibule, les faces intérieures (9) des deux ailettes (5) venant s'appliquer sur les faces vestibulaires des dents respectivement 13 à 14 et 23 à 24 du maxillaire, et
**en ce qu'**il permet en outre l'immobilisation en surélévation de l'occlusion, au moins une partie des molaires droites et gauches du maxillaire s'appuyant par leurs faces occlusales sur la face supérieure (8) de l'appareil (1) lorsque l'appareil (1) est en place en bouche, l'appareil (1) comportant une épaisseur déterminée de matière entre le fond (11) de gouttière et la face supérieure (8) au moins dans les zones où ladite au moins une partie des molaires droites et gauches du maxillaire s'appuient sur la face supérieure de l'appareil afin de bloquer la fermeture et laisser un écart de hauteur déterminé entre les incisives de la mandibule et du maxillaire.

10. Appareil (1) buccodentaire selon la revendication 9, **caractérisé en ce que** la partie antérieure (2) de l'appareil (1) est amincie et la partie antérieure (2) amincie de l'appareil (1) comporte un siège (12) de réception d'un accessoire amovible qui est de préférence un insert (13) de communication aérique interlabiale entre l'intérieur de la cavité buccale et l'environnement extérieur, l'insert (13) comportant une assise (14) en U destinée à venir se positionner à cheval sur le siège (12) et au moins un tube (15) creux destiné à faire communiquer aériquement l'intérieur de la cavité buccale et l'environnement extérieur.

## Patentansprüche

1. Vorrichtung (20) zum Einstellen der Verschlußverhältnisse bei Unterkiefervorschub mit Hilfe von Mitteln zum Einstellen der Vorschubposition des Unterkiefers gegenüber dem Oberkiefer, wobei die Einstellvorrichtung
- eine sich in einem Bogen erstreckende Schale (21) mit einem Querschnitt, der ungefähr die Form eines auf dem Kopf stehenden U hat, und mit einer Innenseite und einer Außenseite, wobei die Schale (21) nach vorne hin einen vorderen Abschnitt und nach hinten hin zwei seitliche Abschnitte, einen rechten und einen linken, aufweist, wobei die Schale (21) dazu ausgelegt ist, mit ihrer Innenseite auf einen Unterkieferbogen eines Patienten deckend aufgelegt zu werden, um einen Abdruck des Unterkieferbogens zu machen, wobei ein oberer Teil der Außenseite gegenüber den Kauflächen von Zähnen des Oberkieferbogens zu liegen kommt,
- mindestens eine hinsichtlich des Vorschubs einstellbare Stützvorrichtung (22), wobei die mindestens eine Stützvorrichtung (22) ein mit der Schale (21) fest verbundenes und zum Abstützen an den Zähnen eines Unterkieferbogens bestimmtes Stützteil (29) aufweist, wobei das Stützteil (29) zum Einstellen des Vorschubs gegenüber der Schale vorwärts oder rückwärts bewegt werden kann, aufweist,
**dadurch gekennzeichnet, daß** die Mittel zum Einstellen der Vorschubposition außerdem ermöglichen, die Vorschubstellung zu speichern, und daß das Stützteil (29) mit dem vorderen Abschnitt der Schale (21) über eine an der Schale (21) festgelegten Gewindestange (32) fest verbunden ist, wobei das Stützteil (29) entlang der Gewindestange (32) gleiten kann und zwischen einer schalenseitigen Feder (31) und einer Mutter (30) auf der Seite des freien Endes der Gewindestange (32) eingeschlossen ist, wobei die Feder (31) das Stützteil (29) gegen die Mutter (30) drückt, wobei die Mutter (30) auf die Gewindestange (32) aufgeschraubt oder von dieser abgeschraubt werden kann, um das Stützteil (29) gegenüber der Schale (21) vorwärts oder rückwärts zu bewegen.

2. Einstellvorrichtung (20) gemäß Anspruch 1, **dadurch gekennzeichnet, daß** das Stützteil (29) eine Y-Form mit zwei auseinanderlaufenden Zweigen und einem gemeinsamen Stamm aufweist, wobei der gemeinsame Stamm des Stützteils (29) mit dem vorderen Abschnitt der Schale fest verbunden ist, wobei einer der Zweige des Y dazu bestimmt ist, an der Vorderseite der Zähne des Unterkieferzahnbogens anzuliegen, und der andere Zweig dazu bestimmt ist, unter der Spitze der Zähne des Unterkieferzahnbogens hindurch zu kommen.

3. Einstellvorrichtung (20) gemäß einem der Ansprüche 1 bis 2. **dadurch gekennzeichnet, daß** sie außerdem Mittel zum Einstellen der Höhe des Verschlusses aufweist, wobei die Einstellvorrichtung außerdem
- mindestens eine hinsichtlich der Öffnung einstellbare Spreizvorrichtung (23) aufweist, wobei die mindestens eine Spreizvorrichtung mit dem oberen Teil der Außenseite eines seitlichen Abschnitts der Schale (21) fest verbunden ist und ein Stützteil (27) aufweist, das dazu bestimmt ist, an den Kauflächen der Zähne eines Unterkieferzahnbogens anzuliegen, wobei das Stützteil (27) zum Einstellen der Öffnung aufwärts oder abwärts bewegt werden kann, wobei die Einstellvorrichtung (20) vorzugsweise zwei hinsichtlich der Öffnung einstellbare Spreizvorrichtungen (23), eine an jedem seitlichen Abschnitt der Schale (21), aufweist.

4. Einstellvorrichtung (20) gemäß Anspruch 3. **dadurch gekennzeichnet, daß** die Mittel zum Einstellen der Höhe des Verschlusses außerdem ermöglichen, die Einstellung der Höhe des Verschlusses zu speichern, und daß die einstellbare Spreizvorrichtung (23) einen auf dem oberen Teil der Außenseite des seitlichen Abschnitts der Schale (21) befestigten Sockel (26) aufweist, wobei der Sockel (26) eine schiefe Ebene bildet, auf der das Stützteil (27) in Form eines Keils gleiten kann, um sich in Abhängigkeit von der Drehrichtung einer Schraube (28) aufwärts oder abwärts zu bewegen.

5. Einstellvorrichtung (20) gemäß einem der Ansprüche 1 bis 4. **dadurch gekennzeichnet, daß** die Schale (21) mindestens einen Durchgang (24) durch den vorderen Teil der Schale zum Steuern der Einstellung des Vorschubs aufweist, das dazu bestimmt ist, das Sichtbarmachen mindestens eines der Schneidezähne des Unterkieferzahnbogens zu ermöglichen, wobei die Schale (21) außerdem an ihrer Außenseite zum oberen Teil der Schale (21) hin und am Rand des Durchgangs (24) eine sich von vorne nach hinten erstreckende Skala (25) aufweist, die dazu bestimmt ist, den Vorschub zwischen den Schneidezähnen des Unterkieferzahnbogens und dem Oberkieferzahnbogen zu messen.

6. Einstellvorrichtung (20) gemäß Anspruch 5, **dadurch gekennzeichnet, daß** die Skala (25) zwischen einer Position, in der sie sich von vorne nach hinten erstreckt, um das Messen des Vorschubs zwischen den Schneidezähnen des Unterkieferzahnbogens und jenen des Oberkieferzahnbogens zu ermöglichen, und einer Position, in der sie senkrecht ausgerichtet ist, um das Messen der Öffnung der Höhe des Verschlusses zwischen den Schneidezähnen des Unterkieferzahnbogens und jenen des Oberkieferzahnbogens zu ermöglichen, kippen kann

7. Einstellvorrichtung (20) gemäß einem der Ansprüche 1 bis 6. **dadurch gekennzeichnet, daß** die Schale mindestens einen Durchgang (24) für eine Auskleidung aufweist, die das Einbringen einer Zahnabdruckpaste ins Innere der Schale auf der Innenseite der Schale ermöglicht.

8. Verfahren zur Herstellung eines Geräts (1) für eine Festlegung des Mund- und Zahnbercichs hinsichtlich des Vorschubs und hinsichtlich der Höhe des Verschlusses des Unterkieferzahnbogens eines Patienten,
**dadurch gekennzeichnet, daß**
- zunächst am Patienten einerseits ein Abdruck des Unterkieferzahnbogens und andererseits ein Abdruck des Oberkieferzahnbogens gemacht wird, wobei der Abdruck des Unterkieferzahnbogens mittels einer Vorrichtung (20) zum Einstellen der Verschlußverhältnisse bei Unterkiefervorschub mit Hilfe von Mitteln (22) zum Einstellen der Vorschubposition des Unterkiefers gegenüber dem Oberkiefer ausgeführt wird und außerdem ermöglicht, die Einstellung des Vorschubs und des Verschlusses für den Patienten zu speichern, wobei die Einstellvorrichtung (20) gemäß Anspruch 5 in Verbindung mit Anspruch 2 ist, und daß beim Machen des Abdrucks des Unterkieferzahnbogens der Vorschub und die Öffnung bei dem Patienten eingestellt und gespeichert werden.
- in einem zweiten Schritt von den Abrücken des Unterkieferzahnbogens und des Oberkieferzahnbogens ein Modell des Unterkieferzahnbogens und ein Modell des Oberkieferzahnbogens hergestellt werden, die beiden Modelle in einen einstellbaren Modellträger (17) eingesetzt werden und der Modellträger (17) eingestellt wird, indem er auf das Modell des Unterkieferzahnbogens gesetzt wird, wobei die Einstellvorrichtung (20) die Einstellung des Vorschubs und der Öffnung eingespeichert hat, um den Modellträger (17) mit derselben Einstellung des Vorschubs und der Öffnung wie beim Patienten einzustellen.
- in einem dritten Schritt, nachdem die Einstellvorrichtung (20) vom Modellträger (17) abgenommen worden ist, das Gerät auf dem in den eingestellten Modellträger eingesetzten Modell hergestellt wird, wobei die Herstellung des Geräts (1) darin besteht, Material auf das in den eingestellten Modellträger eingesetzte Modell des Unterkieferzahnbogenträgers aufzubringen, um eine umgekehrte Rinne (10) zu bilden, die die Zähne des Unterkieferbogens abdeckt, und bei der die Oberseite (8) des Geräts (1) durch Aufbringen einer bestimmten Materialdicke zwischen dem Rinnenboden (11) und der Oberseite (8) mindestens mit einem Teil der Verschlußseiten der rechten und linken Backenzähne des Modells des Unterkieferzahnbogens in Kontakt kommt, und daß außerdem Material auf die Oberseite des Geräts aufgebracht wird, um zwei auf der Oberseite errichtete und sich nach oben erstreckende Flüge! (5) zu bilden, wobei jeder Flügel (5) an den Vorderseiten der Zähne des Modells des Unterkieferzahnbogens, und vorzugsweise an den Zähnen 13 bis 14 und 23 bis 24, anliegt.

9. Gerät (1) für eine Festlegung des Mund- und Zahnbereichs hinsichtlich des Vorschubs des Unterkieferzahnbogens gegenüber dem Oberkieferzahnbogen, das gemäß Anspruch 8 hergestellt ist, wobei das Gerät in Form einer umgekehrten Rinne (10) dazu bestimmt ist, die Zähne des Unterkieferzahnbogens abzudecken, wobei die umgekehrte Rinne (10) nach unten offen, an den Seiten durch eine innere Seitenwand (6) auf der Seite des Munds und eine äußere Seitenwand (7) auf der Seite der Wangen geschlossen und oben durch einen Rinnenboden (11) geschlossen ist, wobei das Gerät (1) drei Hauptteile aufweist, einen nach vorne gerichteten vorderen Teil (2), der dazu bestimmt ist, die Schneidezähne und die Eckzähne des Unterkiefers abzudecken, und einen rechten (4) und einen linken (3) nach hinten gerichteten Seitenteil, die dazu bestimmt sind, die vorderen Backenzähne und die Backenzähne auf der entsprechenden Seite des Unterkiefers abzudecken, wobei die innere Form der Rinne (10) an die Zähne des Unterkiefers angepaßt ist und einem Abdruck der Zähne des Unterkiefers entspricht, wobei das Gerät eine Oberseite (8) aufweist, wobei die Oberseite dazu bestimmt ist, ungefähr gegenüber den Kauflächen der Oberkieferzähne zu liegen zu kommen,
**dadurch gekennzeichnet, daß** das Gerät (1) zwei auf der Oberseite (8) errichtete und nach oben gerichtete Flügel (5) aufweist, wobei jeder Flügel (5) näherungsweise im Anschlußbereich zwischen dem Vorderteil (2) und dem entsprechenden rechten (4) beziehungsweise linken (3) Seitenteil angeordnet ist und sich über eine bestimmte Breite entlang des Unterkieferbogens erstreckt, wobei die Flügel (5) eine zum Inneren des Munds gerichtete Innenseite (9) aufweisen, die dazu bestimmt ist, an den Vorderseiten von Zähnen des Oberkiefers anzuliegen, wenn das Gerät (1) im Mund eingesetzt ist, um den Unterkiefer hinsichtlich des Vorschubs zu blockieren,
wobei die Innenseiten (9) der Flügel (5) an den Vorderseiten der Zähne 13 bis 14 und 23 bis 24 des Unterkiefers anliegen, und
daß es außerdem ein höhenmäßiges Blockieren des Verschlusses ermöglicht, wobei sich mindestens ein Teil der rechten und linken Backenzähne des Oberkiefers mit deren Kauflächen auf der Oberseite (8) des Geräts (1) abstützt, wenn sich das Gerät (1) im Mund befindet, wobei das Gerät mindestens in den Bereichen, in denen ein Teil der rechten und linken Backenzähne des Unterkiefers auf der Oberseite des Geräts aufliegt, um das Verschließen zu blockieren und einen Spalt bestimmter Höhe zwischen den Schneidezähnen des Oberkiefers und des Unterkiefers zu lassen, eine bestimmte Materialdicke zwischen dem Rinnenboden (11) und der Oberseite (8) aufweist.

10. Gerät für den Mund- und Zahnbereich (1) gemäß Anspruch 9, **dadurch gekennzeichnet, daß** der vordere Teil (2) des Geräts (1) schmal ist und der schmale Teil (2) des Geräts (1) einen Sitz (12) zum Aufnehmen eines abnehmbaren Zubehörs aufweist, das vorzugsweise ein Einsatz (13) für die luftmäßige interlabiale Verbindung zwischen dem Inneren der Mundhöhle und der äußeren Umgebung ist, wobei der Einsatz (13) einen U-förmigen Sitz (14), der dazu bestimmt ist, sich rittlings auf dem Sitz (12) zu positionieren, und mindestens ein Hohlrohr (15), das dazu bestimmt ist, das Innere der Mundhöhle mit der äußeren Umgebung luftmäßig zu verbinden, aufweist.

## Claims

1. A device for adjusting the mandibular protrusion occlusion ratios (20) with means for adjusting the protrusion positioning of the mandible with respect to the maxilla, the device including:
- an elongated arched shell (21) having an approximately inverted U-shaped cross-section with an inner face and an outer face, said shell (21) having an anterior portion on the front and two right and left side portions on the rear, said shell (21) being adapted to be placed with its inner face covering a mandibular dental arch of a subject for taking an impression of said mandibular dental arch, an upper part of the outer face coming opposite occlusal faces of teeth of the maxillary dental arch,
- at least one adjustable protrusion-adjustment pressing device (22), said at least one pressing device (22) including a pressing element (29) integral with the anterior portion of the shell (21) and intended to come into abutment against teeth of a maxillary dental arch, the pressing element (29) being able to be move forward or rearward with respect to the shell to adjust the protrusion,
**characterized in that** the means for adjusting the protrusion positioning further allow the protrusion adjustment to be recorded and **in that** the pressing element (29) is made integral with the anterior portion of the shell (21) by means of a threaded rod (32) immobilized to the shell (21), said pressing element (29) being able to slide along said threaded rod (32) and being taken in sandwich between a spring (31) on the shell side and a nut (30) on the free end side of the threaded rod (32), the spring (31) pushing the pressing element (29) against the nut (30), said nut (30) being able to be screwed on or unscrewed from the threaded rod (32) to move the pressing element (29) forward or rearward with respect to the shell (21).

2. The adjustment device (20) according to claim 1, **characterized in that** the pressing element (29) is Y-shaped with two diverging arms and a common trunk, said common trunk of the pressing element (29) being integral with the anterior portion of the shell, one arm of the Y being intended to come into abutment against the front face of the teeth of the maxillary dental arch and the other arm being intended to pass under the tip of the teeth of the maxillary dental arch.

3. The adjustment device (20) according to any one of claims 1 to 2, **characterized in that** it further comprises means for adjusting the occlusion elevation, the adjustment device further including:
- at least one adjustable spacing device (23) for adjusting the opening, said at least one spacing device being integral to the upper part of the outer face of a side portion of the shell (21) and including a support element (27) intended to come against occlusal faces of teeth of a maxillary dental arch, the support element (27) being able to be moved up or down for adjusting the opening, preferably the adjustment device (20) including two adjustable opening-adjustment spacing devices (23), one on each side portion of the shell (21).

4. The adjustment device (20) according to claim 3, **characterized in that** the means for adjusting the occlusion elevation further allow the occlusion elevation adjustment to be recorded and **in that** the adjustable spacing device (23) includes a base (26) fastened to the upper part of the outer face of the side portion of the shell (21), said base (26) forming an inclined plane on which the wedge-shaped support element (27) may slide to move up or down according to the direction of rotation of a screw (28).

5. The adjustment device (20) according to any one of claims 1 to 4, **characterized in that** the shell (21) includes at least one passage (24) through the anterior portion of the shell to control the protrusion adjustment and intended to allow the visualisation of at least one of the incisors of the mandibular dental arch, the shell (21) further including at its outer face, towards the top of the shell (21) and at the edge of said passage (24), a graduated scale (25) that is elongated in the anteroposterior direction and that is intended to measure the protrusion progression of the incisors of the mandibular dental arch and of the maxillary dental arch.

6. The adjustment device (20) according to claim 5, **characterized in that** the graduated scale (25) can tilt between a position in which it is elongated in the anteroposterior direction to allow the measurement of the protrusion progression between the incisors of the mandibular dental arch and of the maxillary dental arch and a position in which it is elongated in the vertical direction to allow the measurement of the opening of the occlusion elevation between the incisors of the mandibular dental arch and of the maxillary dental arch.

7. The adjustment device (20) according to any one of claims 1 to 6, **characterized in that** the shell includes at least one filling passage (24) for introducing an impression taking paste into the shell, on the inner face side of the shell.

8. A method for manufacturing an oral appliance (1) for immobilizing the mandibular arch in protrusion and the occlusion in elevation for a subject, **characterized in that**:
- firstly, on the one hand, an impression of the maxillary dental arch and, on the other hand, an impression of the mandibular dental arch, are made on the subject, the maxillary dental arch impression taking being made with an adjustment device (20) provided means for adjusting the protrusion positioning (22) and for adjusting the mandible opening (23) with respect to the maxilla and further allowing the protrusion and opening adjustment to be recorded, said adjustment device (20) being according to claim 5 in its combination with claim 2, and during the mandibular dental arch impression taking, the protrusion and the opening are adjusted and recorded for the subject,
- secondly, a maxillary dental arch model and a mandibular dental arch model are made from the maxillary and mandibular dental arch impressions, the two models are installed in an adjustable model-holder (17) and said model-holder (17) is adjusted by placing on the mandibular dental arch model the adjustment device (20) having recorded the protrusion and opening adjustment in order to configure the model-holder (17) with the same protrusion and opening adjustment as on the subject,
- thirdly, the adjustment device (20) having been removed from the model-holder (17), the appliance is made on the mandibular dental arch model installed in the configured model-holder, the making of the appliance (1) consisting in applying material on the mandibular dental arch model installed in the configured model-holder in such a way as to form an inverted gutter (10) covering the teeth of the mandibular arch and whose upper face (8) of the appliance (1) comes into contact with at least one occlusal face part of the right and left molars of the maxillary dental arch model by application of a determined thickness of material between the gutter bottom (11) and said upper face (8), and **in that**, moreover, material is applied to said upper face of the appliance so as to form two fins (5) erected on said upper side and extended upwards, each fin (5) coming into abutment against vestibular faces of teeth of the maxillary dental arch model, and preferably against teeth 13 to 14 and 23 to 24, respectively.

9. An oral appliance (1) for immobilizing the mandibular arch in protrusion with respect to the maxillary arch made according to the method of claim 8, said appliance having the shape of an inverted gutter (10) being intended to cover the teeth of the mandibular arch, said inverted gutter (10) being open downwards, closed on the sides by internal side walls (6) on the inner side of the mouth and external side walls (7) on the cheek side and closed upwards by a gutter bottom (11), said appliance (1) including three main portions, an anterior portion (2) towards the front, intended to cover mandibular incisors and canines and two right (4) and left (3) side portions towards the rear, intended to cover the premolar and molar teeth on the corresponding side of the mandible, the inner shape of the gutter (10) being adjusted to the teeth of the mandible and corresponding to an impression of said mandible teeth, the appliance including an upper face (8), said upper face being intended to come approximately opposite the occlusal faces of the maxillary teeth,
**characterized in that** the appliance (1) includes two fins (5) erected on the upper face (8) and extended upwards, each fin (5) being positioned approximately in the area of connection between the anterior portion (2) and the corresponding right (4) or left (3) side portion, and extending along the mandibular arch over a determined width, the fins (5) including an inner face (9) directed towards the interior of the mouth and intended to come into abutment against the vestibular faces of maxillary teeth when the appliance (1) is in place in the mouth in order to block the mandible in protrusion, the inner faces (9) of the two fins (5) being applied against the vestibular faces of teeth 13 to 14 and 23 to 24, respectively, and
**in that** it further allows immobilizing the occlusion in elevation, at least part of the right and left molars of the maxilla pressing by their occlusal faces on the upper face (8) of the appliance (1) when the appliance (1) is in place in the mouth, the appliance (1) having a determined thickness of material between the gutter bottom (11) and the upper face (8) at least in the areas in which said at least part of the right and left molars of the maxilla presses on the upper face of the appliance in order to block the closure and leave a determined height gap between the incisors of the mandible and of the maxilla.

10. The oral appliance (1) according to claim 9, **characterized in that** the anterior portion (2) of the appliance (1) is thinned and the thinned anterior portion (2) of the appliance (1) includes a seat (12) for receiving a removable accessory that is preferably an insert (13) for aerial interlabial communication between the interior of the oral cavity and the external environment, the insert (13) including a U-shaped basis (14) intended to come astride the seat (12) and at least one hollow tube (15) for the aerial communication of the interior of the oral cavity with the external environment.
